# EUROPÄISCHE PATENTANMELDUNG

(11) **EP 3 534 376 A1**
(43) Veröffentlichungstag der Anmeldung: **04.09.2019**
(21) Anmeldenummer: 18159242.9
(22) Anmeldetag: 28.02.2018
(51) Int. Cl.: G21K 1/02, G21K 1/06

(54) **VERFAHREN ZUR HERSTELLUNG EINES MIKROSTRUKTURBAUTEILS, MIKROSTRUKTURBAUTEIL UND RÖNTGENGERÄT**

(71) Anmelder: Siemens Healthcare GmbH, 91052 Erlangen (DE)
(72) Erfinder: DEUTINGER, Andrea, 85659 Forstern (DE)

(57) **Zusammenfassung**

Bei einem Verfahren zur Herstellung eines Mikrostrukturbauteils (4), das insbesondere als Röntgenphasenkontrastgitter in einem Röntgengerät (1) zum Einsatz kommt, wird verfahrensgemäß in eine zumindest um eine Biegeachse deformierbare Form (26), die durch ein Siliziumsubstrat gebildet ist, und die eine Vielzahl von in einer Dickenrichtung (10) des Siliziumsubstrats (26) verlaufenden Aussparungen (38) mit Abmessungen im Mikrometerbereich aufweist, ein Röntgenstrahlen absorbierendes Material eingefüllt. Die Form (26) mit dem eingefüllten Material wird auf einen oberhalb der Raumtemperatur und unterhalb eines Schmelztemperaturwerts des eingefüllten Materials liegenden Arbeitstemperaturwert temperiert und in eine bestimmungsgemäße Endkontur verformt.

## Beschreibung

Die vorliegende Erfindung betrifft ein Verfahren zur Herstellung eines Mikrostrukturbauteils, insbesondere eines Röntgenphasenkontrastgitters. Des Weiteren betrifft die vorliegende Erfindung ein Mikrostrukturbauteil, insbesondere ein Röntgenphasenkontrastgitter sowie ein Röntgengerät mit einem solchen.

Im Bereich der Röntgenbildgebung insbesondere im medizinischen Bereich, wird teilweise der sogenannte Talbot-Effekt genutzt. Hierdurch können präzisere Bildinformationen generiert werden, indem der Kontrast der erzeugten Bilder durch Einbeziehung einer von einem Untersuchungsobjekt verursachten Phasenverschiebung der Röntgenstrahlen verbessert wird. Dabei kommt regelmäßig ein sogenanntes (Röntgen-)Phasenkontrastgitter zum Einsatz, das in den Strahlengang eingebracht wird. Ein solches Gitter ist dabei üblicherweise durch in Röntgen-Strahlungsrichtung ausgerichtete Lamellen aus einem röntgenstrahlenabsorbierenden Material gebildet. Üblicherweise liegen die Wandstärke und die Abstände dieser Lamellen zueinander dabei im ein- bis gering zweistelligen Mikrometerbereich. Auch die Dicke (oder: Höhe) des gesamten Gitters liegt im durchstrahlten Bereich bei meist maximal einem Millimeter. Somit handelt es sich bei einem solchen Phasenkontrastgitter um ein Mikrostrukturbauteil.

Aufgrund der geringen Abmessungen werden diese Phasenkontrastgitter meist durch Ätzverfahren in Siliziumwafern und nachträglichem Füllen der geätzten Vertiefungen mit Röntgenstrahlen absorbierendem Material, insbesondere einem Metall oder einer Metalllegierung, zur Ausbildung der Lamellen hergestellt. Dies ist beispielsweise aus DE 10 2015 201 741 A1 bekannt. Problematisch ist dabei häufig, dass die Phasenkontrastgitter idealerweise an die lokale Strahlungsrichtung der von einer meist punktförmigen Strahlungsquelle ausgehenden Radialstrahlen angepasst sein sollten. D. h. die Lamellen sollten idealerweise in einem Winkel zueinander angestellt sein.

Der Erfindung liegt die Aufgabe zugrunde, ein verbessertes Mikrostrukturbauteil zu ermöglichen.

Diese Aufgabe wird erfindungsgemäß gelöst durch ein Verfahren zur Herstellung eines solchen Mikrostrukturbauteils mit den Merkmalen des Anspruchs 1. Des Weiteren wird diese Aufgabe erfindungsgemäß gelöst durch ein Mikrostrukturbauteil mit den Merkmalen des Anspruchs 13. Außerdem wird die Aufgabe erfindungsgemäß gelöst durch ein Röntgengerät mit den Merkmalen des Anspruchs 14. Vorteilhafte und teils für sich erfinderische Ausführungsformen und Weiterbildungen der Erfindung sind in den Unteransprüchen und der nachfolgenden Beschreibung dargelegt.

Das erfindungsgemäße Verfahren dient zur Herstellung eines Mikrostrukturbauteils, insbesondere eines (Röntgen-)Phasenkontrastgitters. Verfahrensgemäß wird dabei in eine zumindest um eine Biegeachse (insbesondere elastisch) deformierbare Form, die durch ein Siliziumsubstrat gebildet ist und die eine Vielzahl von in einer Dickenrichtung des Siliziumsubstrats verlaufenden Aussparungen aufweist, ein Röntgenstrahlen absorbierendes Material eingefüllt. Die Aussparungen weisen dabei Abmessungen im Mikrometerbereich auf (d. h. insbesondere in Flächenrichtung gesehen kleinste Abmessungen betragen etwa bis 10 Mikrometer, insbesondere etwa 0,5, 1 oder bis zu 4 Mikrometer). Die Form mit dem eingefüllten Material wird auf einen oberhalb der Raumtemperatur und unterhalb eines Schmelztemperaturwerts des eingefüllten Materials liegenden Arbeitstemperaturwert temperiert und anschließend in eine bestimmungsgemäße Endkontur verformt.

Dadurch, dass die Verformung der mit dem Material gefüllten Form unterhalb der Schmelztemperatur des Materials erfolgt, wird vorteilhafterweise verhindert, dass das Material schmilzt und im schmelzflüssigen Zustand aus den Aussparungen austreten kann. Des Weiteren ist eine Füllung der Form, konkret der Aussparungen vor der Verformung in die Endkontur regelmäßig einfacher als die Füllung einer bereits vorgeformten Form.

Besonders bevorzugt wird die "gefüllte" Form, d. h. die Form mit dem eingefüllten Material bei dem Arbeitstemperaturwert unter (insbesondere temperatur-unterstütztem) Kriechen des eingefüllten Materials auf die bestimmungsgemäße Endkontur verformt. Unter Kriechen (oder auch: "Retardation") wird dabei insbesondere zumindest eine Gefügeänderung verstanden, die vergleichsweise langsam und meist unter vergleichsweise geringer Belastung abläuft und zu einer plastischen Verformung führt. Meist sind die Vorgänge, die zu einer solchen Gefügeänderung führen, thermisch aktiviert, erfolgen (insbesondere bei Metallen) also erst ab einer meist gegenüber Raumtemperatur erhöhten Temperatur. Durch die vorstehend beschriebene Ausnutzung des Kriechens des eingefüllten Materials wird vorteilhafterweise ermöglicht, die gefüllte Form mit vergleichsweise geringen Umformkräften in die Endkontur zu verformen. Dadurch kann vorteilhafterweise ein Risiko, die Mikrostrukturen des Mikrostrukturbauteils und/oder der Form zu beschädigen und somit die Genauigkeit des Mikrostrukturbauteils zu verringern, gesenkt werden.

Vorzugsweise wird als Röntgenstrahlen absorbierendes Material, insbesondere ein Metall (vorzugsweise mit einer vergleichsweise hohen Kriechneigung), beispielsweise Zinn oder eine Zinnlegierung herangezogen.

In einer bevorzugten Verfahrensvariante wird als Arbeitstemperaturwert ein Wert von etwa 20-50 Prozent, insbesondere von etwa 30-40 Prozent des Schmelztemperaturwerts des Röntgenstrahlen absorbierenden Materials herangezogen. Insbesondere handelt es sich bei dem Arbeitstemperaturwert mindestens um den Wert einer sogenannten Übergangstemperatur, ab der Gefügemechanismen des Materials (insbesondere des Metalls) thermisch aktiviert ablaufen. Ab diesem Temperaturwert nimmt somit die Kriechneigung insbesondere metallischer Materialien zu. Regelmäßig erfolgt dabei eine plastische Deformation bereits bei in das Material eingebrachten Spannungen (Spannungswerten), die unterhalb einer Streckgrenze des Materials liegen. Besonders bevorzugt wird dabei das Röntgenstrahlen absorbierende Material, insbesondere das Metall oder eine Metalllegierung derart gewählt, dass die Deformation durch Kriechen bei einem Arbeitstemperaturwert und Spannungswerten erfolgt, die vorteilhafter Weise nicht zu einer Schädigung der Struktur der Form führen. Somit kann die Form mit dem eingefüllten Röntgenstrahlen absorbierenden Material vorteilhafterweise mit derart geringen Belastungen umgeformt werden, die bei unterhalb dem Arbeitstemperaturwert liegenden Temperaturwerten, insbesondere bei Raumtemperatur lediglich zu einer elastischen Verformung führen würden. Bei Zinn liegt der Arbeitstemperaturwert insbesondere bei etwa 200 Grad Celsius.

In einer zweckmäßigen Verfahrensvariante wird das Röntgenstrahlen absorbierende Material in schmelzflüssigem Zustand in die Form, insbesondere in die Aussparungen eingebracht. Insbesondere kommt dabei ein Druckgussverfahren zum Einsatz. Durch das schmelzflüssige Einfüllen des Materials wird vorteilhafterweise eine mechanische Wechselwirkung zwischen der Form und dem einzufüllenden Material verringert, sodass wiederum das Risiko, die Mikrostrukturen der Form zu beschädigen, verringert wird. Insbesondere durch die Nutzung von Druck wird dabei eine möglichst vollständige Füllung der Form und somit eine präzise Abformung der Mikrostrukturen ermöglicht.

In einer besonders zweckmäßigen Verfahrensvariante wird das Siliziumsubstrat der Form vor dem Einbringen des Röntgenstrahlen absorbierenden Materials zur Vermeidung einer chemischen Bindung des Materials mit dem Siliziumsubstrat vorbehandelt. Beispielsweise wird das Siliziumsubstrat - vorzugsweise alle Oberflächen, die bestimmungsgemäß mit dem einzuführenden Material in Kontakt kommen - konditioniert (beispielsweise mit einer Lösung, Plasma oder dergleichen) oder beschichtet (beispielsweise mittels Sputtern, PVD, CVD oder dergleichen). Dadurch wird ermöglicht, dass die Form in einem optionalen Verfahrensschritt nach (oder alternativ auch vor) dem Umformen zumindest teilweise entfernt werden kann, sodass das in die Aussparungen eingefüllte Material beispielsweise als Säule, Lamelle oder dergleichen zumindest zum Großteil vereinzelt stehen bleibt.

In einer weiteren zweckmäßigen Verfahrensvariante wird die Form mit dem eingefüllten Material mittels eines Werkzeugs verformt, das eine Unterschale und eine Oberschale mit jeweils gegengleich entlang einer Kreiszylinderfläche gewölbten Formflächen aufweist (die Formflächen sind also jeweils in die gleiche Richtung gewölbt und verlaufen im bestimmungsgemäßen Einsatzzustand somit parallel zueinander). Insbesondere wird die gefüllte Form zwischen die Unterschale und die Oberschale eingelegt und die Oberschale anschließend mit einer zur "Kriech-Umformung" erforderlichen Kraft belastet.

Vorzugsweise wird zur Verformung insbesondere mittels der vorstehend beschriebenen Oberschale eine Kraft (auch als "Schließkraft" bezeichnet) auf die Form ausgeübt, die insbesondere einer Masse von 10-150 Gramm, vorzugsweise von 20-100 Gramm entspricht. Eine solche Kraft ist insbesondere bei Abmessungen des Mikrostrukturbauteils in Dickenrichtung von kleiner als ein Millimeter, insbesondere im Bereich bis etwa 500 Mikrometer zur Kriech-Umformung unter Vermeidung von Beschädigungen der Mikrostrukturen der Form ausreichend.

In einer fertigungstechnisch zweckmäßigen Verfahrensvariante wird die Verformung der gefüllten Form in einem Ofen, insbesondere einem Umluftofen, durchgeführt, in dem die Temperierung auf den Arbeitstemperaturwert stattfindet. Vorzugsweise wird dabei die gefüllte Form in das vorstehend beschriebene Werkzeug eingelegt, mit der entsprechenden Kraft belastet und anschließend in den Ofen eingestellt. In dem Ofen erwärmen sich das Werkzeug und somit auch die eingelegte Form auf den Arbeitstemperaturwert, sodass aufgrund der aufgebrachten Kraft das Röntgenstrahlen absorbierende Material sich durch Kriechen insbesondere auf die durch das Werkzeug vorgegebene Endkontur verformt. Vorzugsweise ist die Form dabei derart elastisch ausgebildet, dass sie der Verformung des eingefüllten Materials elastisch folgt. Durch das Kriechen des eingefüllten Materials findet aber eine plastische Verformung des Materials statt, wodurch auch die Form selbst nach dem Entformen aus dem Werkzeug die erreichte Endkontur beibehält.

In einer bevorzugten Verfahrensvariante werden die Aussparungen der Form durch eine ätztechnische Ausbildung von Spalten gebildet. Diese Spalte verlaufen dabei in einer ersten Substratrichtung und sind durch Querstege, die in einer zur ersten Substratrichtung senkrecht stehenden zweiten Substratrichtung verlaufen, voneinander getrennt. Außerdem sind die Spalte auch zeilenartig in der zweiten Substratrichtung nebeneinander angeordnet, wobei die einzelnen Zeilen durch parallel zu den Spalten verlaufende Längsstege voneinander getrennt sind. D. h. die Spalte sind in einem Raster angeordnet, in dem innerhalb der Zeilen die Spalte mit ihrer Längsrichtung parallel zur ersten Substratrichtung stehen und in der zweiten Substratrichtung mehrere Zeilen (insbesondere mit jeweils mehreren Spalten) nebeneinander angeordnet sind. Vorzugsweise ist dabei das gesamte Siliziumsubstrat zumindest in der ersten Substratrichtung von dem Raster abgedeckt. Aufgrund dieser Ausrichtung der Spalte wird eine Biegesteifigkeit des Siliziumsubstrats (und somit der Form) um eine in der ersten Substratrichtung ausgerichtete Biegeachse vorteilhaft verringert. Vorzugsweise erfolgt die Umformung der (gefüllten) Form auch um diese Biegeachse.

In einer besonders zweckmäßigen Weiterbildung sind die vorstehend beschriebenen Spalte, die in der zweiten Substratrichtung aufeinanderfolgen, bezüglich ihrer Längserstreckung entlang der ersten Substratrichtung zueinander versetzt angeordnet. D. h. die Spalte sind von Zeile zu Zeile in der ersten Substratrichtung verschoben. Vorzugsweise sind die Spalte dabei um die Hälfte ihrer bestimmungsgemäßen Längserstreckung verschoben. Dadurch ergibt sich, dass die vorstehend beschriebenen Querstege in der zweiten Substratrichtung gesehen nur in jeder zweiten Zeile miteinander fluchten. Außerdem ergibt sich durch die Verschiebung um die halbe Längserstreckung eine gleichmäßige und insbesondere symmetrische Deformation bei einer Biegung um die vorstehend beschriebene Biegeachse.

In einer weiteren zweckmäßigen Verfahrensvariante werden die Aussparungen, insbesondere die vorstehend beschriebenen Spalte insbesondere mittels anisotroper Ätzverfahren derart ausgeformt, dass sie das Siliziumsubstrat in Dickenrichtung durchdringen. Mithin sind die Aussparungen bzw. Spalte vorzugsweise beidseitig zu dem Siliziumsubstrat geöffnet.

Vorzugsweise wird zur Ausbildung der Form in eine erste Oberfläche des insbesondere scheibenartigen Siliziumsubstrats (bspw. in einen Wafer oder einen Teil eines Wafers) insbesondere ätztechnisch eine Vielzahl von punktartigen (vorzugsweise pyramidenförmigen) und insbesondere voneinander separaten Impfstrukturen in dem vorstehend beschriebenen Raster eingebracht. Die Impfstrukturen werden dabei in der ersten Substratrichtung in Gruppen zusammengefasst, die der jeweiligen Aussparung, vorzugsweise einem jeden Spalt zugeordnet sind. Die Gruppen sind in der ersten Substratrichtung voneinander zur späteren Ausbildung der Querstege beabstandet. Darauf folgend werden die Impfstrukturen in Tiefenrichtung (oder auch: Dickenrichtung) des Siliziumsubstrats in einem Ätzschritt zu Bohrlöchern verlängert - d. h. vertieft. Anschließend wird eine der ersten Oberfläche des Siliziumsubstrats gegenüberliegende zweite Oberfläche zur rückseitigen Öffnung der Bohrlöcher in einem weiteren Ätzschritt zumindest teilweise entfernt. In einem nachfolgenden Ätzschritt wird dann ein anisotrop wirksames Ätzmedium - insbesondere eine Ätzlösung - alternierend von beiden Oberflächen des Siliziumsubstrats her durch die Bohrlöcher gespült, so dass sich die (insbesondere zunächst runden) Bohrlöcher (vorzugsweise zu einem quadratischen Querschnitt) aufweiten und sich innerhalb der jeweiligen Gruppe zu der jeweiligen Aussparung, insbesondere zu dem in der ersten Substratrichtung verlaufenden Spalt verbinden. Vorzugsweise wird die erste Oberfläche des Siliziumsubstrats dabei derart gewählt, dass sie parallel zu einer "(100)-Kristallebene" des Siliziums ausgerichtet ist.

In einer bevorzugten Verfahrensvariante werden die Aussparungen, insbesondere die Spalte mit einem Aspektverhältnis in Dickenrichtung des Siliziumsubstrats von etwa 1:450 ausgeformt. Die Aussparungen bzw. Spalte weisen dabei insbesondere in der zweiten Substratrichtung gesehen Abmessungen (d. h. eine Breite) von etwa ein bis vier Mikrometern auf. Derart "feine" (d. h. kleine und filigrane) Strukturen sind insbesondere für den Einsatz des Mikrostrukturbauteils als (Röntgen-) Phasenkontrastgitter in einem Röntgengerät vorteilhaft. Die zur Absorption von Röntgenstrahlen dienenden Lamellen dieses Phasenkontrastgitters werden dabei durch das Röntgenstrahlen absorbierende und in die Aussparungen eingefüllte Material gebildet. Vorzugsweise liegen die vorstehend beschriebenen, durch die in der ersten Substratrichtung verlaufenden Spalte gebildeten Zeilen dabei mit einem Abstand von etwa 2-12 Mikrometern auseinander. Dies entspricht insbesondere einer sogenannten Gitterkonstanten des Phasenkontrastgitters.

Weiter vorzugsweise werden die vorstehend beschriebenen Spalte mit einer Längserstreckung zwischen 20 und 1300, vorzugsweise zwischen 50 und weniger als 1000 Mikrometern ausgebildet. Insbesondere bei Werten unter 1000 (insbesondere bis zu 300) Mikrometern ergeben sich einerseits eine zur Umformung der gefüllten Form hinreichend geringe aber zur Handhabung (auch der gefüllten Form) ausreichend hohe Biegesteifigkeit insbesondere um eine in der ersten Substratrichtung liegende Biegeachse.

Um die Biegesteifigkeit der gefüllten Form für das Umformen um diese Biegeachse weiter zu senken, wird in einem optionalen Verfahrensschritt vor dem Umformen in einem (weiteren) Ätzschritt ein zentraler Bereich der Rückseite (d. h. der zweiten Oberfläche) des Siliziumsubstrats bis auf eine Restdicke entfernt und nur in der zweiten Substratrichtung endständige Randbereiche des Siliziumsubstrats belassen. In dem zentralen Bereich stehen dabei - wie vorstehend beschrieben - die durch das eingefüllte Material gebildeten Säulen oder Lamellen aus dem Siliziumsubstrat vor. Insbesondere für den Einsatz des Mikrostrukturbauteils als Phasenkontrastgitter wird in dem zentralen Bereich (der vorzugsweise einem durchstrahlten Bereich entspricht) dadurch auch eine Absorption der Röntgenstrahlen im Silizium verringert.

Das erfindungsgemäße Mikrostrukturbauteil stellt insbesondere das vorstehend genannte Phasenkontrastgitter dar und ist gemäß dem vorstehend beschriebenen Verfahren hergestellt. D. h. das Mikrostrukturbauteil weist ebenfalls die vorstehend beschriebenen, insbesondere sich aus dem Herstellungsverfahren ergebenden körperlichen Merkmale und Vorteile auf.

Das erfindungsgemäße Röntgengerät weist das durch das vorstehend beschriebene Mikrostrukturbauteil gebildete Phasenkontrastgitter auf, und teilt somit ebenfalls die vorstehend beschriebenen Merkmale und Vorteile.

Nachfolgend werden Ausführungsbeispiele der Erfindung anhand einer Zeichnung näher dargestellt. Darin zeigen:
- FIG 1: in einer schematischen Seitenansicht ein Röntgengerät mit einem Phasenkontrastgitter,
- FIG 2: in einem schematischen Ablaufdiagramm ein Verfahren zur Herstellung des Phasenkontrastgitters,
- FIG 3: in einer schematischen Draufsicht ein Raster von Impfstrukturen zur Erzeugung des Phasenkontrastgitters aus einem Siliziumsubstrat,
- FIG 4: in Ansicht gemäß FIG 3 aus dem Raster gebildete Spalte in dem Siliziumsubstrat,
- FIG 5: in einer schematischen Schnittansicht das Phasenkontrastgitter in einem Fertigungszwischenschritt, und
- FIG 6,7: in jeweils unterschiedlichen schematischen Perspektivansichten ein Werkzeug zur Umformung des Phasenkontrastgitters auf eine Endkontur.

Einander entsprechende Teile sind in allen Figuren stets mit gleichen Bezugszeichen versehen

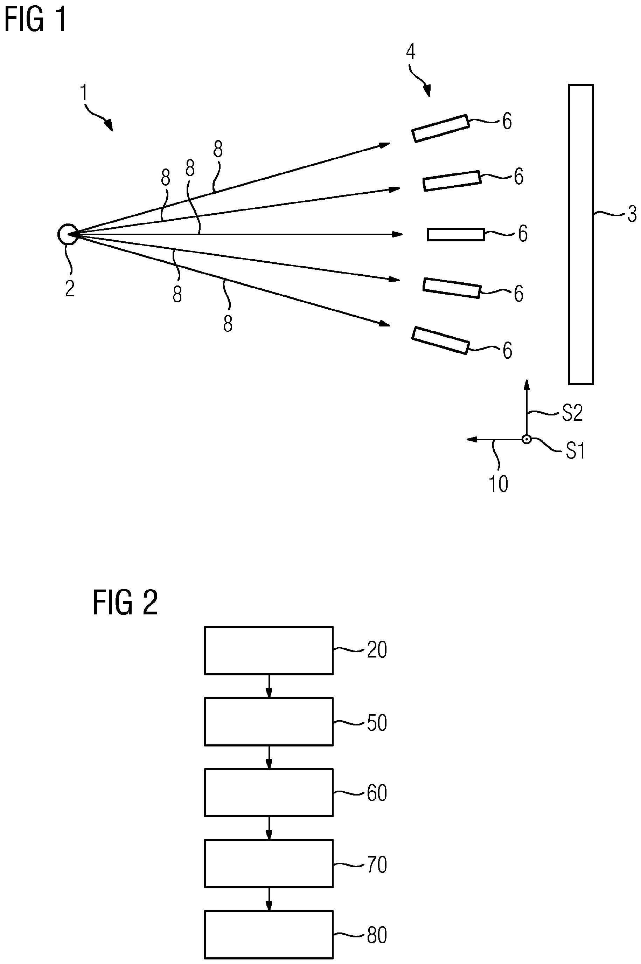

In FIG 1 ist schematisch angedeutet ein Röntgengerät 1 dargestellt. Das Röntgengerät 1 weist eine Röntgen-Strahlenquelle 2 und einen Röntgen-Strahlendetektor 3 auf. Im Strahlengang zwischen der Röntgen-Strahlenquelle 2 und dem Röntgen-Strahlendetektor 3 ist ein (Röntgen-) Phasenkontrastgitter 4 angeordnet. Das Phasenkontrastgitter 4 weist dabei eine Anzahl von Lamellen 6 auf, die parallel zu dem jeweiligen lokalen Röntgen-Teilstrahl 8 ausgerichtet sind. Das Phasenkontrastgitter 4 weist dabei in Dickenrichtung 10 (oder auch: entlang der Strahlungsrichtung) gesehen Abmessungen von maximal einem Millimeter auf. Somit handelt es sich bei dem Phasenkontrastgitter 4 um ein Bauteil mit Strukturen im Mikrometerbereich ("Mikrostrukturbauteil"). Um bei solchen geringen Abmessungen dennoch die Lamellen 6 des Phasenkontrastgitters 4 präzise abformen zu können, wird ein im Folgenden anhand von FIG 2 näher beschriebenes Herstellungsverfahren durchgeführt.

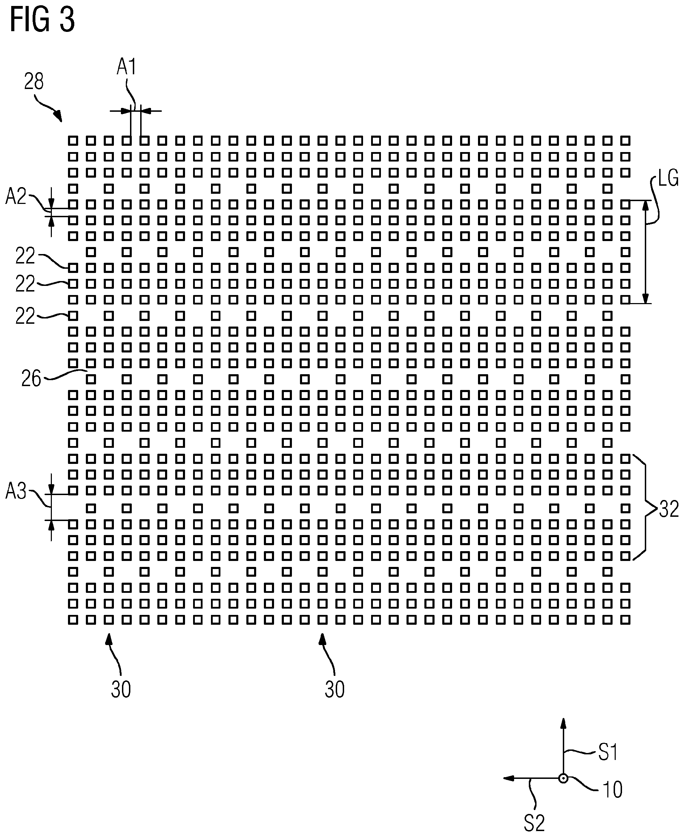

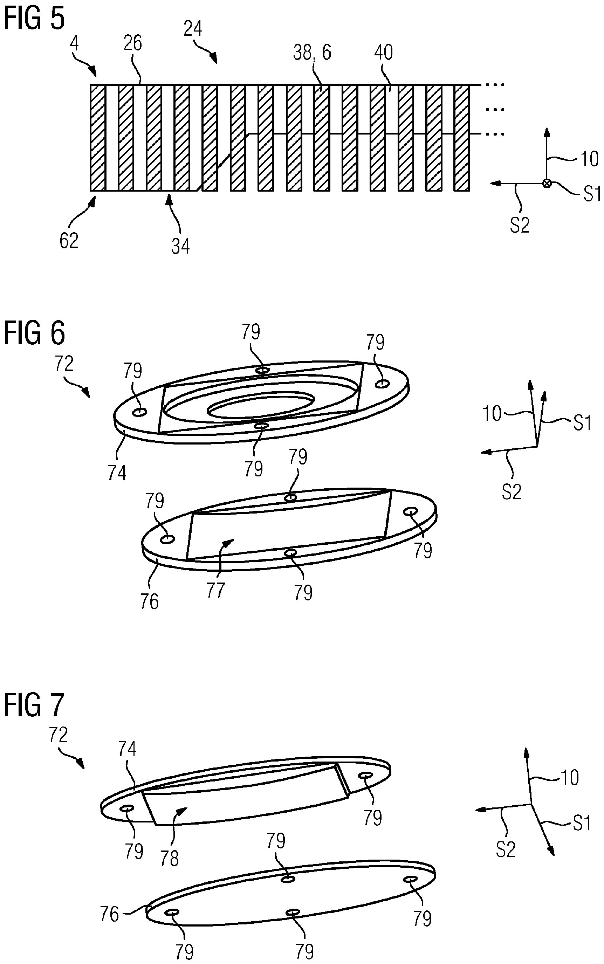

In einem ersten Verfahrensschritt 20 werden dabei Impfstrukturen 22 (s. FIG 3) in eine erste Oberfläche 24 (s. FIG 5) eines scheibenartigen Siliziumsubstrats 26 (mit einer Ausgangsdicke von mehr als 500 Mikrometern) eingebracht. Bei dem Siliziumsubstrat 26 handelt es sich konkret um einen Silizium-Wafer. Die Impfstrukturen 22 sind dabei in einem Raster 28 über die erste Oberfläche 24 des Siliziumsubstrats 26 verteilt. Das Raster 28 ist dabei (konkret zeilenartig) in einer ersten Substratrichtung S1 und einer zweiten Substratrichtung S2 vorgegeben. D. h. die Impfstrukturen 22 werden in Zeilen 30, die in der ersten Substratrichtung S1 verlaufen und sich in der zweiten Substratrichtung S2 wiederholen, angeordnet. In Zeilenrichtung - d. h. in der ersten Substratrichtung S1 - sind jeweils mehrere Impfstrukturen 22 (im vorliegenden Ausführungsbeispiel konkret sieben Impfstrukturen 22) zu jeweils einer Gruppe 32 zusammengefasst. Die jeweiligen Zeilen 30, konkret die in der zweiten Substratrichtung S2 nebeneinander angeordneten Impfstrukturen 22 werden dabei mit einem ersten Abstand A1 zueinander angeordnet. Der erste Abstand A1 beträgt im vorliegenden Ausführungsbeispiel 12 Mikrometer und entspricht dabei konkret einer Gitterkonstanten des Phasenkontrastgitters 4. Innerhalb der Gruppen 32 sind die Impfstrukturen 22 mit einem zweiten Abstand A2 zueinander angeordnet, der um den Faktor 0,5 kleiner ist als der erste Abstand A1.

Die Impfstrukturen 22 sind dabei quadratisch mit einer Kantenlänge von kleiner oder gleich 2 Mikrometern ausgebildet. Eine Längserstreckung LG der Gruppen 32 beträgt somit etwa 50 Mikrometer.

Die Gruppen 32 sind außerdem mit einem dritten Abstand A3 zueinander beanstandet angeordnet. Der dritte Abstand A3 entspricht dabei etwa einer Auslassung einer der Impfstrukturen 22 in der jeweiligen Zeile 30.

Die Impfstrukturen 22 werden anschließend in Dickenrichtung 10 mittels eines Ätzverfahrens zu Bohrlöchern erweitert, deren Seitenwände senkrecht zu der ersten Oberfläche 24 stehen. Dazu wird das sogenannte PAECE-Verfahren ("photo assisted electro-chemical etching") eingesetzt. Konkret werden die Impfstrukturen 22 zu kreiszylindrischen Bohrlöchern mit einer Tiefe von 470 Mikrometern erweitert.

Die der ersten Oberfläche 24 gegenüberliegende zweite Oberfläche 34 wird anschließend derart entfernt, dass die Bohrlöcher rückseitig (d. h. zu der zweiten Oberfläche 34 hin) geöffnet sind. Dazu kommen Ätzverfahren wie zum Beispiel nasschemisches Ätzen mit Kaliumhydroxid oder plasmainduziertes Trockenätzen zum Einsatz. In einem im bestimmungsgemäßen Einsatzzustand im Röntgengerät 1 von den Röntgen-Teilstrahlen 8 durchstrahlten Bereich beträgt die Dicke des Siliziumsubstrats 26 anschließend 450 Mikrometer.

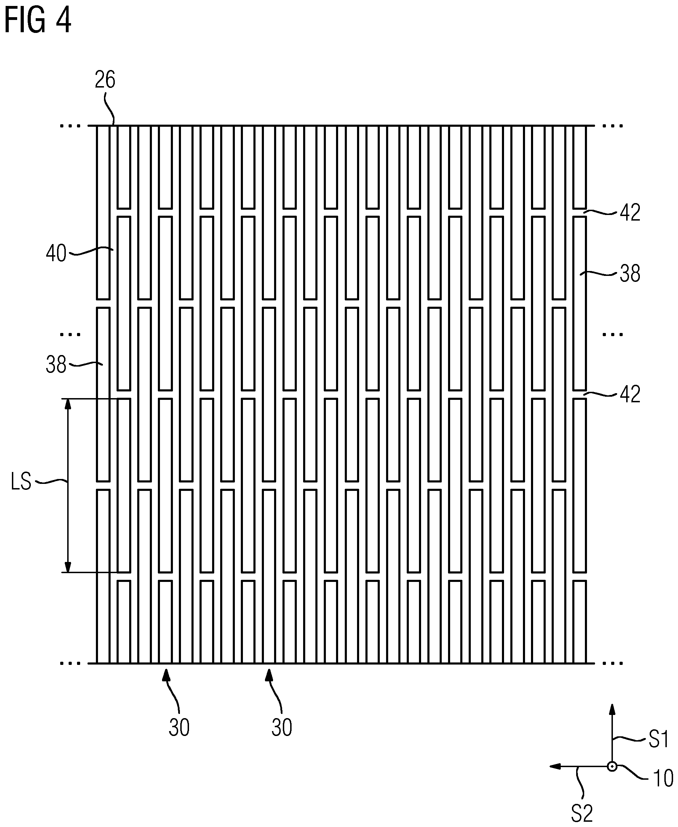

In einem weiteren Ätzschritt werden die Bohrlöcher alternierend von der ersten Oberfläche 24 und der zweiten Oberfläche 34 her mit einem anisotrop wirksamen Ätzmedium durchspült. Das Ätzmedium enthält dabei Kaliumhydroxid, Wasserstoffperoxid und Isopropanol. Die erste und die zweite Substratrichtung S1 bzw. S2 sind parallel zu Richtungen gewählt, in denen (111)-Kristallebenen des Siliziums durch die (parallel zur ersten Oberfläche 24 liegenden) (100)-Kristallebene hindurch treten. Dadurch werden die einzelnen Bohrlöcher in der ersten und zweiten Substratrichtung S1 und S2 aufgeweitet, so dass sie einen quadratischen Querschnitt aufweisen, und vereinigen sich bei fortschreitender Aufweitung mit den benachbarten Bohrlöchern innerhalb der jeweiligen Gruppen 32 zu jeweils einem Spalt 38 (s. FIG 4, 5). Da der erste Abstand A1 größer ist als der zweite Abstand A2 verbleiben nach der Spaltbildung zwischen den einzelnen Zeilen 30 Längsstege 40 aus Silizium. Aufgrund des dritten Abstands A3 zwischen den einzelnen Gruppen 32 verbleibt auch in der ersten Substratrichtung S1 gesehen zwischen den durch die Gruppen 32 gebildeten Spalten 38 jeweils ein Quersteg 42.

Das Raster 28 ist dabei derart gewählt, dass die gebildeten Spalte 38 zweier direkt benachbarter Zeilen 30 um die Hälfte ihrer Längserstreckung LS in der ersten Substratrichtung S1 versetzt sind. Dadurch fluchten die Querstege 42 zweier direkt benachbarter Zeilen 30 nicht miteinander. Vielmehr fluchten die Querstege 42 immer der "übernächsten" Zeilen 30 miteinander. Dadurch ergibt sich ein guter Kompromiss von mechanischer Stabilität gegen eine Biegung um eine in der ersten Substratrichtung S1 gerichtete Biegeachse und einer hinreichenden Flexibilität für eine solche Biegung.

In einem zweiten Verfahrensschritt 50 wird das Siliziumsubstrat 26, konkret die Oberflächen 24 und 34 sowie die Innenwände der Spalte 38 vorbehandelt, um eine chemische Bindung eines in einem nachfolgenden Verfahrensschritt 60 in die Spalte 38 einzufüllenden Röntgenstrahlen absorbierenden Materials, konkret einer Zinnlegierung, mit den Innenwänden und den Oberflächen 24 bzw. 34 zu verhindern.

In dem Verfahrensschritt 60 wird dazu nachfolgend mittels eines Druckgussverfahrens die Zinnlegierung in die Spalte 38 zur Ausbildung der Lamellen 6 eingefüllt. Das Siliziumsubstrat 26 bildet somit eine Form, in die die Zinnlegierung eingebracht wird.

Wie aus FIG 5 ersichtlich ist, werden in einem optionalen Teilschritt des Verfahrensschritts 60 nach dem Einfüllen der Zinnlegierung in einem weiteren Ätzschritt (in einem zusätzlichen, optionalen Verfahrensschritt) zentrale Bereiche der Rückseite (d. h. auf der Seite der zweiten Oberfläche 34) des Siliziumsubstrats 26 in Dickenrichtung 10 teilweise entfernt. Nur in der zweiten Substratrichtung S2 verbleiben endständige Randbereiche 62 (auch: Seitenbereiche), so dass in diesen Randbereichen 62 eine massivere und damit mechanisch stabilere Handlingstruktur ("Griffstruktur") vorhanden ist. Konkret werden die Randbereiche 72 vor dem Ätzen entsprechend maskiert. Das Ätzen erfolgt mittels Kaliumhydroxid. Die durch die Zinnlegierung in den gefüllten Spalten 38 gebildeten Lamellen 6 stehen hiernach zumindest teilweise frei. Dadurch wird in die Biegesteifigkeit des gefüllten Siliziumsubstrats 26 um die in der ersten Substratrichtung S1 verlaufende Biegeachse weiter verringert.

Optional erfolgt im Rahmen des Verfahrensschritts 60 auch ein Zuschnitt des Siliziumsubstrats 26 auf die für das Röntgengerät 1 erforderlichen Abmessungen (nicht näher dargestellt, optional vor der Ausbildung der Randbereiche 62). Dabei werden die erste und die zweite Substratrichtung S1 bzw. S2 berücksichtigt und gegebenenfalls Markierungen zur Justierung aufgebracht. Der Zuschnitt wird bspw. mittels Laserstrahlschneiden oder Wafersägen durchgeführt.

In einem weiteren Verfahrensschritt 70 wird das mit der Zinnlegierung gefüllte Siliziumsubstrat 26 in ein Werkzeug 72 eingelegt. Das Werkzeug 72 ist in FIG 6, 7 näher dargestellt und weist eine Oberschale 74 und eine Unterschale 76 auf. An der Unterschale 76 sind eine untere Formfläche 77 und an der Oberschale 74 eine obere Formfläche 78 ausgearbeitet. Die beiden Formflächen 77 bzw. 78 sind dabei gegengleich entlang einer Kreiszylinder-Mantelfläche gewölbt. Konkret ist die untere Formfläche 77 konkav in die Unterschale 76 eingearbeitet. Entsprechend ist die obere Formfläche 78 konvex von der Oberschale 74 vorstehend ausgebildet. Der Wölbungsradius der beiden Formflächen 77 bzw. 78 beträgt dabei 300 Millimeter. Das gefüllte Siliziumsubstrat 26 wird dabei derart in das Werkzeug 72 eingelegt, dass die erste Substratrichtung S1 parallel zur Zylinderachse der Formflächen 77 bzw. 78 ausgerichtet ist. Dadurch erfolgt die Biegung des Siliziumsubstrats 26 um die in der ersten Substratrichtung S1 ausgerichtete Biegeachse.

Die Oberschale 74 wird nach dem Schließen des Werkzeugs 72 mit Gewichtsstücken oder Federn derart gegen die Unterschale 76 "verspannt", dass sich eine Schließkraft, die einer Masse von 50 Gramm entspricht, ergibt.

Die Oberschale 74 und die Unterschale 76 sind aus einer Aluminiumlegierung gefertigt. Zum Schließen des Werkzeugs 72 werden die Oberschale 74 und die Unterschale 76 mittels Führungsstiften, die in korrespondierende Bohrungen 79 der Oberschale 74 und der Unterschale 76 eingesetzt werden, zueinander geführt.

In einem nachfolgenden Verfahrensschritt 80 wird das geschlossene Werkzeug 72 (mit dem darin eingelegten, gefüllten Siliziumsubstrat 26) in einen auf 200 Grad Celsius temperierten Ofen eingestellt. Bei diesem Temperaturwert (auch als "Arbeitstemperaturwert" bezeichnet) tritt unter der Schließkraft des Werkzeugs 72 in der Zinnlegierung Kriechen (auch als "Retardation" bezeichnet) auf. D. h. die Zinnlegierung durchläuft trotz der geringen Schließkraft eine plastische Verformung. Aufgrund der vorstehend beschriebenen Rasterung des Siliziumsubstrats 26 mit den Spalten 38 ist die Biegesteifigkeit des Siliziumsubstrats 26 derart gering, dass sich das Siliziumsubstrat 26 unter Kriechen der Zinnlegierung an die Formflächen 77 bzw. 78 anlegt. Aufgrund der plastischen Verformung der Zinnlegierung wird die durch die Formflächen 77 bzw. 78 vorgegebene Kontur auch nach Öffnen des Werkzeugs 72 von dem mit der Zinnlegierung gefüllten Siliziumsubstrat 26 als Endkontur beibehalten.

Durch die vorstehend beschriebene Verformung sind die durch die mit der Zinnlegierung gefüllten Spalte 38 gebildeten Lamellen 6 rotationssymmetrisch ausgerichtet, sodass eine periodische, homogene Röntgenabsorption, die dem Strahlungsfeld - konkret dem jeweiligen Röntgen-Teilstrahl 8 - angeglichen ist, möglich ist. Außerdem liegen die bestimmungsgemäßen Schließkräfte des Werkzeugs 72 außerhalb eines Bereichs, in dem die Längsstege 40 und/oder die Querstege 42 mechanisch geschädigt werden würden. Somit erfolgt bei der vorstehend beschriebenen Kriech-Umformung keine Kompression der Lamellen 6 sondern nur die rotationssymmetrische Ausrichtung um die erste Substratrichtung S1. Letzteres kann optional dadurch unterstützt werden, dass in den Randbereichen 62 die Längsstege 40 (und somit auch die Querstege 42) bis zur rückseitigen Oberfläche 34 vorstehen.

In einem optionalen, nicht näher dargestellten weiteren Verfahrensschritt wird das Siliziumsubstrat 26 zumindest zu einem Großteil entfernt, sodass die Lamellen 6 "kammartig" stehen bleiben. Beispielsweise verbleibt in Dickenrichtung 10 gesehen ein Rest des Siliziumsubstrats 26, der im Vergleich zur in Dickenrichtung 10 verlaufenden "Höhe" der Lamellen 6 kurz ist, um den geometrischen Zusammenhalt der vereinzelten Lamellen 6 zu ermöglichen. Dieser Verfahrensschritt erfolgt insbesondere, wenn die teilweise Entfernung in dem in FIG 5 beschriebenen Teilschritt nicht stattgefunden hat. In einem weiteren Ausführungsbeispiel erfolgt dieser Verfahrensschritt aber auch zusätzlich, um die Lamellen 6 noch weiter frei zu legen.

Abschließend wird das durch die Lamellen 6 gebildete Phasenkontrastgitter 4 in das Röntgengerät 1 eingebaut.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

## Patentansprüche

1. Verfahren zur Herstellung eines Mikrostrukturbauteils (4), wobei verfahrensgemäß
- in eine zumindest um eine Biegeachse deformierbare Form (26), die durch ein Siliziumsubstrat gebildet ist, und die eine Vielzahl von in einer Dickenrichtung (10) des Siliziumsubstrats (26) verlaufenden Aussparungen (38) mit Abmessungen im Mikrometerbereich aufweist, ein Röntgenstrahlen absorbierendes Material eingefüllt wird,
- die Form (26) mit dem eingefüllten Material auf einen oberhalb der Raumtemperatur und unterhalb eines Schmelztemperaturwerts des eingefüllten Materials liegenden Arbeitstemperaturwert temperiert wird, und
- die Form (26) mit dem eingefüllten Material in eine bestimmungsgemäße Endkontur verformt wird.

2. Verfahren nach Anspruch 1,
wobei die Form (26) unter Kriechen des eingefüllten Materials in die bestimmungsgemäße Endkontur verformt wird.

3. Verfahren nach Anspruch 1 oder 2,
wobei als Arbeitstemperaturwert ein Wert von etwa 20 bis 50 Prozent, insbesondere von etwa 30 bis 40 Prozent des Schmelztemperaturwerts herangezogen wird.

4. Verfahren nach einem der Ansprüche 1 bis 3,
wobei das Röntgenstrahlen absorbierende Material in schmelzflüssigem Zustand in die Form, insbesondere die Aussparungen (38) eingebracht wird.

5. Verfahren nach einem der Ansprüche 1 bis 4,
wobei die Form (26) vor dem Einbringen des Röntgenstrahlen absorbierenden Materials zur Vermeidung einer chemischen Bindung des Röntgenstrahlen absorbierenden Materials mit dem die Form bildenden Siliziumsubstrat (26) vorbehandelt wird.

6. Verfahren nach einem der Ansprüche 1 bis 5,
wobei die Form (26) mit dem eingefüllten Material mittels eines Werkzeugs (72), das eine Unterschale (76) und eine Oberschale (74) mit jeweils gegengleich entlang einer Kreiszylinderfläche gewölbten Formflächen (77,78) aufweist, verformt wird.

7. Verfahren nach einem der Ansprüche 1 bis 6,
wobei zur Verformung eine einer Masse von 10 bis 150 Gramm entsprechende Kraft auf die Form (26) ausgeübt wird.

8. Verfahren nach einem der Ansprüche 1 bis 7,
wobei die Verformung in einem Ofen unter Temperierung auf den Arbeitstemperaturwert durchgeführt wird.

9. Verfahren nach einem der Ansprüche 1 bis 8,
wobei die Aussparungen der Form (26) durch ätztechnische Ausbildung von Spalten (38), die in einer ersten Substratrichtung (S1) verlaufen und durch Querstege (42) voneinander getrennt und die zeilenartig parallel zueinander in einer zweiten, zur ersten Substratrichtung (S1) senkrecht stehenden Substratrichtung (S2) angeordnet sind, gebildet werden.

10. Verfahren nach Anspruch 9,
wobei die Spalte (38), die in der zweiten Substratrichtung (S2) aufeinander folgen, bezüglich ihrer Längserstreckung (LS) entlang der ersten Substratrichtung (S1) zueinander versetzt angeordnet werden.

11. Verfahren nach einem der Ansprüche 1 bis 10,
wobei die Aussparungen (38) derart ausgeformt werden, dass sie das Siliziumsubstrat (26) in Dickenrichtung (10) durchdringen.

12. Verfahren nach einem der Ansprüche 1 bis 11,
wobei die Aussparungen (38) ein Aspektverhältnis in Dickenrichtung (10) von etwa 1:450 aufweisen, und wobei die Aussparungen (38) eine Breite von 1 bis 4 Mikrometern aufweisen.

13. Mikrostrukturbauteil (4), insbesondere Röntgenphasenkontrastgitter hergestellt nach einem Verfahren nach einem der Ansprüche 1 bis 12.

14. Röntgengerät (1) mit einem durch das Mikrostrukturbauteil nach Anspruch 13 gebildeten Röntgenphasenkontrastgitter (4) .

## Geänderte Patentansprüche

### Geänderte Patentansprüche gemäss Regel 137(2) EPÜ.

1. Verfahren zur Herstellung eines Mikrostrukturbauteils (4), wobei verfahrensgemäß
- in eine zumindest um eine Biegeachse deformierbare Form (26), die durch ein Siliziumsubstrat gebildet ist, und die eine Vielzahl von in einer Dickenrichtung (10) des Siliziumsubstrats (26) verlaufenden Aussparungen (38) mit Abmessungen im Mikrometerbereich aufweist, ein Röntgenstrahlen absorbierendes Material in schmelzflüssigem Zustand eingefüllt wird,
- die Form (26) mit dem eingefüllten Material auf einen oberhalb der Raumtemperatur und unterhalb eines Schmelztemperaturwerts des eingefüllten Materials liegenden Arbeitstemperaturwert temperiert wird, und
- die Form (26) mit dem eingefüllten Material in eine bestimmungsgemäße Endkontur verformt wird.

2. Verfahren nach Anspruch 1,
wobei die Form (26) unter Kriechen des eingefüllten Materials in die bestimmungsgemäße Endkontur verformt wird.

3. Verfahren nach Anspruch 1 oder 2,
wobei als Arbeitstemperaturwert mindestens der Wert einer sogenannten Übergangstemperatur, ab der Gefügemechanismen des Materials thermisch aktiviert ablaufen, herangezogen wird.

4. Verfahren nach einem der Ansprüche 1 bis 3,
wobei das Röntgenstrahlen absorbierende Material in die Aussparungen (38) der Form (26) eingebracht wird.

5. Verfahren nach einem der Ansprüche 1 bis 4,
wobei die Form (26) vor dem Einbringen des Röntgenstrahlen absorbierenden Materials zur Vermeidung einer chemischen Bindung des Röntgenstrahlen absorbierenden Materials mit dem die Form bildenden Siliziumsubstrat (26) vorbehandelt wird.

6. Verfahren nach einem der Ansprüche 1 bis 5,
wobei die Form (26) mit dem eingefüllten Material mittels eines Werkzeugs (72), das eine Unterschale (76) und eine Oberschale (74) mit jeweils gegengleich entlang einer Kreiszylinderfläche gewölbten Formflächen (77,78) aufweist, verformt wird.

7. Verfahren nach einem der Ansprüche 1 bis 6,
wobei zur Verformung eine einer Masse von 10 bis 150 Gramm entsprechende Kraft auf die Form (26) ausgeübt wird.

8. Verfahren nach einem der Ansprüche 1 bis 7,
wobei die Verformung in einem Ofen unter Temperierung auf den Arbeitstemperaturwert durchgeführt wird.

9. Verfahren nach einem der Ansprüche 1 bis 8,
wobei die Aussparungen der Form (26) durch ätztechnische Ausbildung von Spalten (38), die in einer ersten Substratrichtung (S1) verlaufen und durch Querstege (42) voneinander getrennt und die zeilenartig parallel zueinander in einer zweiten, zur ersten Substratrichtung (S1) senkrecht stehenden Substratrichtung (S2) angeordnet sind, gebildet werden.

10. Verfahren nach Anspruch 9,
wobei die Spalte (38), die in der zweiten Substratrichtung (S2) aufeinander folgen, bezüglich ihrer Längserstreckung (LS) entlang der ersten Substratrichtung (S1) zueinander versetzt angeordnet werden.

11. Verfahren nach einem der Ansprüche 1 bis 10,
wobei die Aussparungen (38) derart ausgeformt werden, dass sie das Siliziumsubstrat (26) in Dickenrichtung (10) durchdringen.

12. Verfahren nach einem der Ansprüche 1 bis 11,
wobei die Aussparungen (38) ein Aspektverhältnis in Dickenrichtung (10) von etwa 1:450 aufweisen, und wobei die Aussparungen (38) eine Breite von 1 bis 4 Mikrometern aufweisen.

13. Mikrostrukturbauteil (4), insbesondere Röntgenphasenkontrastgitter hergestellt nach einem Verfahren nach einem der Ansprüche 1 bis 12, aufweisend eine zumindest um eine Biegeachse deformierbare Form (26), die durch ein Siliziumsubstrat gebildet ist, und die eine Vielzahl von in einer Dickenrichtung (10) des Siliziumsubstrats (26) verlaufenden Aussparungen (38) mit Abmessungen im Mikrometerbereich aufweist, wobei in Flächenrichtung gesehen kleinste Abmessungen etwa bis 10 Mikrometer betragen, wobei ein Röntgenstrahlen absorbierendes Material in die Form (26) eingefüllt ist, wobei das Röntgenstrahlen absorbierende Material in schmelzflüssigem Zustand in die Form eingebracht ist, und wobei die Form (26) mit dem eingefüllten Material in eine bestimmungsgemäße Endkontur verformt ist.

14. Röntgengerät (1) mit einem durch das Mikrostrukturbauteil nach Anspruch 13 gebildeten Röntgenphasenkontrastgitter (4) .
